# EUROPÄISCHE PATENTSCHRIFT

(11) **EP 1 969 990 B1**
(45) Veröffentlichungstag und Bekanntmachung des Hinweises auf die Patenterteilung: **18.11.2009**
(21) Anmeldenummer: 08002500.0
(22) Anmeldetag: 12.02.2008
(51) Int. Cl.: A61B 1/00, G02B 23/24

(54) **Kupplungsmechanismus**
Coupling mechanism
Mécanisme d'embrayage

(30) Priorität: 21.02.2007 DE 102007008422
(43) Veröffentlichungstag der Anmeldung: 17.09.2008
(73) Patentinhaber: Karl Storz GmbH & Co. KG, 78532 Tuttlingen (DE)
(72) Erfinder: Frey, Sebastian, 78054 Villingen-Schwenningen (DE); Sauer, Michael, 78532 Tuttlingen (DE)
(74) Vertreter: Hofmeister, Frank

(56) Entgegenhaltungen:
- DE-A1- 3 014 116
- DE-A1- 4 425 705
- DE-C1- 19 712 645

## Beschreibung

Die Erfindung betrifft einen Kupplungsmechanismus, insbesondere für medizinische Instrumente, mit einem Kupplungsstecker und einer Kupplungsaufnahme zur Aufnahme des Kupplungssteckers, wobei die Kupplungsaufnahme und der Kupplungsstecker über mindestens eine federbelastete Rastverbindung aneinander festlegbar sind, wobei die Rastverbindung über einen Sicherungsmechanismus aktivierbar ist, die über den in die Kupplungsaufnahme eingesteckten Kupplungsstecker freigebbar ist.

Patentschrift DE 197 12 645 C1 offenbart eine Kupplung zum Kuppeln zweier optischer Einrichtungen.

Ein gattungsgemäßer Kupplungsmechanismus ist beispielsweise aus der DE 44 25 705 C2 bekannt. Dieser bekannte Kupplungsmechanismus ist Bestandteil eines endoskopischen Instruments, wobei die Kupplungsaufnahme am proximalen Ende des Instruments ausgebildet ist und der Kupplungsstecker Bestandteil eines zweiten, mit diesem endoskopischen Instrument zu verbindenden Instruments ist. Dieser bekannte Kupplungsmechanismus besteht aus einer Bajonettkupplung sowie einem federbelasteten Rastelement, über das die beiden Bauteile gegeneinander fixierbar sind. Dieser bekannte Kupplungsmechanismus hat sich in der Praxis durchaus bewährt, jedoch ist der Aufbau mit einer Bajonettkupplung und einem Rastelement sehr aufwendig. Darüber hinaus bedarf es einer gesonderten manuellen Verdrehung eines Drehrings, um die beiden Bauteile gegeneinander zu fixieren.

Davon ausgehend liegt der Erfindung die **Aufgabe** zugrunde, einen Kupplungsmechanismus der eingangs genannten Art zu schaffen, der bei einfacher Handhabung eine sichere Verrastung der miteinander zu verbindenden Bauteile gewährleistet.

Die **Lösung** dieser Aufgabenstellung ist erfindungsgemäß dadurch gekennzeichnet, dass der Sicherungsmechanismus als in der Kupplungsaufnahme angeordnetes federbelastetes Rastelement ausgebildet ist.

Durch die erfindungsgemäße Ausbildung des erfindungsgemäßen Sicherungsmechanismus als in der Kupplungsaufnahme angeordnetes federbelastetes Rastelement wird einerseits ein versehentliches Verriegeln verhindert und andererseits eine automatische Verriegelung des eingesetzten Kupplungssteckers gewährleistet, wenn die Rastverbindung durch Betätigung des Sicherungsmechanismus aktiviert wurde. Diese Ausführungsform hat den Vorteil, dass das Aktivieren der Rastverbindung und das Koppeln der Bauteile Kupplungsstecker und Kupplungsaufnahme in zeitlicher Abfolge in einem Arbeitsgang erfolgen kann, wodurch das Verbinden der Bauteile einfach und sicher zu handhaben ist.

Gemäß einer praktischen Ausführungsform der Erfindung besteht das Rastelement des erfindungsgemäßen Sicherungsmechanismus aus einem axial verschiebbaren Stift und einem mit dem Stift in Wirkverbindung stehenden radial verschiebbaren Klemmelement, wobei das Klemmelement über ein Federelement in die den Sicherungsmechanismus sperrende Richtung vorgespannt ist. Durch die Federvorspannung des Klemmelements des Sicherungsmechanismus wird sichergestellt, dass die Rastverbindung deaktiviert bleibt und somit ein versehentliches Verriegeln verhindert, solange nicht zuvor der Sicherungsmechanismus sachgemäß betätigt wurde.

Um das Zusammenwirken des axial verschiebbaren Stifts mit dem radial verschiebbaren Klemmelement zu erleichtern, wird mit der Erfindung vorgeschlagen, dass am Stift und am Klemmelement miteinander korrespondierende Anlaufschrägen ausgebildet sind.

Weiterhin wird mit einer bevorzugten Ausführungsform der Erfindung vorgeschlagen, dass die Rastverbindung aus mindestens einem an der Kupplungsaufnahme angeordneten und über mindestens ein Federelement in Verrastungsrichtung federbelasteten Rasthaken sowie mindestens einer am Kupplungsstecker angeordneten Rastaufnahme zur Aufnahme des mindestens einen Rasthakens besteht. Durch die erfindungsgemäße Ausgestaltung des Kupplungsmechanismus ist es möglich, nach dem Aktivieren der Rastverbindung durch Betätigen des Sicherungsmechanismus die beiden miteinander zu verbindenden Bauteile durch bloßes Zusammenstecken gas- und flüssigkeitsdicht miteinander zu verbinden. Einer zusätzlichen manuellen Tätigkeit oder eines Werkzeugeinsatzes bedarf es bei dem erfindungsgemäßen Mechanismus nicht.

Gemäß einer praktischen Ausführungsform der Erfindung umfasst die Kupplungsaufnahme eine Innenhülse und eine die Innenhülse zumindest teilweise koaxial umgebende Außenhülse, wobei die Innenhülse und die Außenhülse über ein Federelement, insbesondere eine Drucktorsionsfeder, gegeneinander verdrehbar miteinander gekoppelt sind. Das Federelement dient dabei einerseits dem axialen Toleranzausgleich zwischen dem in die Kupplungsaufnahme einzusetzenden Kupplungsstecker und den Rastelementen der Rastverbindung und andererseits erlaubt das Federelement eine gegenseitige Verdrehbarkeit der Innen- und Au-βenhülse gegeneinander.

Zur Ausbildung der erfindungsgemäßen Rasthaken sind an den Rasthaken nach innen weisende Rastnasen ausgebildet, die im mit dem Kupplungsstecker verrasteten Zustand als Stege ausgebildete Rastaufnahmen am Kupplungsstecker hintergreifen. Das Koppeln und Entkoppeln der Rasthaken der Kupplungsaufnahme mit den Rastaufnahmen am Kupplungsstecker kann erfindungsgemäß dadurch erleichtert werden, dass die Rasthaken radial verschiebbar in der Außenhülse gelagert sind und im mit dem Kupplungsstecker verrasteten Zustand innenseitig an eine Anlagefläche der Innenhülse anliegen.

Zum Trennen der beiden über den erfindungsgemäßen Kupplungsmechanismus miteinander verbundenen Bauteile ist die Rastverbindung über einen mit dem mindestens einen Rasthaken in Wirkverbindung stehenden Auslösemechanismus wieder aufhebbar.

Gemäß einer praktischen Ausführungsform zur Ausbildung des Auslösemechanismus wird vorgeschlagen, dass zur Ausbildung des Auslösemechanismus die Anlagefläche der Innenhülse, an der die Rasthaken im verrasteten Zustand anliegen, als unrunde Kulissenbahn ausgebildet ist. Durch die gegenseitige Verdrehbeweglichkeit der Innenhülse und der Außenhülse zueinander, verfahren die Rasthaken beim gegenseitigen Verdrehen der Bauteile entlang dieser Kulissenbahn und können aufgrund der unrunden Ausbildung der Kulissenbahn radial nach außen innen verschoben werden, bis die Rastnasen die Stege des Kupplungssteckers und die Innenhülse wieder freigeben und durch die Federwirkung Innenhülse und Außenhülse auseinander gedrückt werden.

Weiterhin wird mit der Erfindung vorgeschlagen, dass an der Außenhülse radial nach außen vorstehende Nocken ausgebildet sind. Durch diese Nocken kann die Handhabung der Kupplungsaufnahme, insbesondere das gegenseitige Verdrehen der Bauteile Innenhülse und Außenhülse, erleichtert werden, da sie wie Handgriffe das Ergreifen der Außenhülse erleichtern.

Schließlich wird mit der Erfindung vorgeschlagen, dass am Kupplungsstecker und an der Kupplungsaufnahme miteinander korrespondierende Führungs- und/oder Zentrierelemente ausgebildet sind, um eine lagegenaue und verdrehsichere Platzierung der Bauteile zueinander zu gewährleisten und folglich eine schnelle und einwandfreie Kopplung über die Rastverbindung zu ermöglichen.

Weiterhin betrifft die Erfindung eine Kupplungsaufnahme zur Aufnahme eines Kupplungssteckers eines Kupplungsmechanismus, insbesondere für medizinische Instrumente, wobei die Kupplungsaufnahme und der Kupplungsstecker über mindestens eine federbelastete Rastverbindung aneinander festlegbar sind. Da der Kupplungsstecker bei praktischen Ausführungsformen derartiger medizinischer Kupplungsmechanismen häufig an einem vielseitig verwendbaren Standard Instrument, beispielsweise einer Endoskop-Optik, ausgebildet ist, die über eine Kupplungsaufnahme mit verschiedenen anderen medizinischen Instrumenten koppelbar ist, zeichnet sich die erfindungsgemäße Kupplungsaufnahme dadurch aus, dass die Rastverbindung über einen Sicherungsmechanismus aktivierbar ist, um so bei einfacher Handhabung eine sichere, lagestabile und gas- und flüssigkeitsdichte Verrastung der Bauteile zu gewährleisten.

Weitere Merkmale und Vorteile der Erfindung ergeben sich anhand der zugehörigen Zeichnung, in der ein Ausführungsbeispiel eines erfindungsgemäßen Kupplungsmechanismus nur beispielhaft dargestellt ist, ohne die Erfindung auf dieses Ausführungsbeispiel zu beschränken. In der Zeichnung zeigt:
- Fig. 1: eine Explosionszeichnung eines erfindungsgemäßen Kupplungsmechanismus;
- Fig. 2: eine Draufsicht auf den zusammengesetzten Kupplungsmechanismus gemäß Fig. 1 im unverrasteten Zustand, jedoch ohne Abdeckkappe und Kupplungsstecker;
- Fig. 3: eine perspektivische Seitenansicht des Kupplungsmechanismus gemäß Fig. 2, jedoch mit Kupplungsstecker, aber ohne Außenhülse;
- Fig. 4: eine Ansicht gemäß Fig. 2, jedoch ohne Abdeckkappe; jedoch den Kupplungsmechanismus im verrasteten Zustand darstellend;
- Fig. 5: eine perspektivische Seitenansicht des Kupplungsmechanismus gemäß Fig. 4, jedoch mit Kupplungsstecker, aber ohne Außenhülse;
- Fig. 6: einen Längsschnitt entlang der Linie VI-VI gemäß Fig. 2, jedoch mit Kupplungsstecker;
- Fig. 7: einen Längsschnitt entlang der Linie VII-VII gemäß Fig. 2, jedoch mit Kupplungsstecker und Abdeckkappe;
- Fig. 8: einen Längsschnitt entlang der Linie VIII-VIII gemäß Fig. 4, jedoch mit Kupplungsstecker;
- Fig. 9: einen Längsschnitt entlang der Linie IX-IX gemäß Fig. 4, jedoch mit Kupplungsstecker und Abdeckkappe
- Fig. 10: eine vergrößerte Ansicht des Details X gemäß Fig. 6 in der sperrenden und freigebenden Stellung;
- Fig.11: eine perspektivische Seitenansicht des zusammengesetzten Kupplungsmechanismus gemäß Fig. 1 im unverrasteten Zustand, jedoch ohne Abdeckkappe und Außenhülse und
- Fig. 12: eine Ansicht gemäß Fig. 11, jedoch den Kupplungsmechanismus im verrasteten Zustand darstellend.

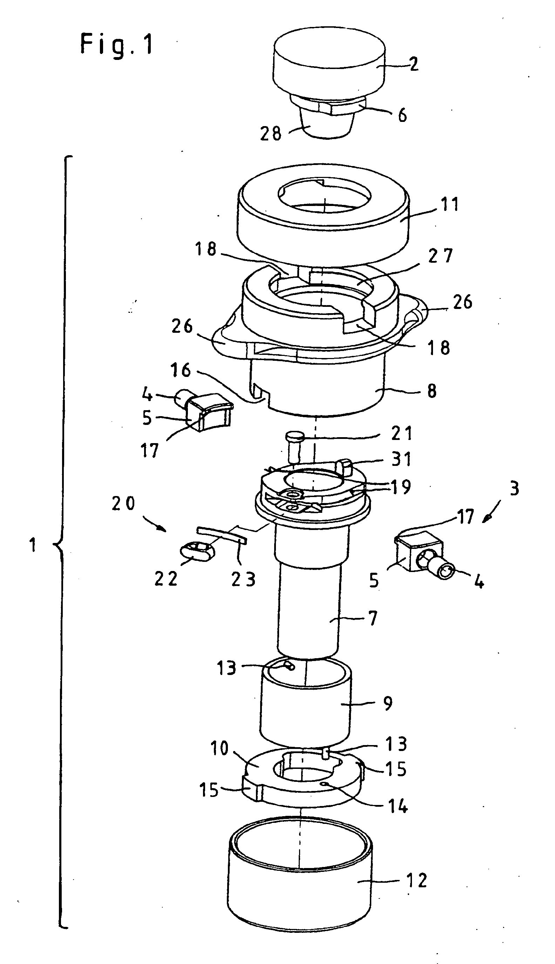

Der in den Abbildungen Fig. 1 bis Fig. 12 dargestellte Kupplungsmechanismus besteht im Wesentlichen aus einer Kupplungsaufnahme 1 und einem in der Kupplungsaufnahme 1 über eine Rastverbindung 3 verrastend festlegbaren Kupplungsstecker 2, wobei die Rastverbindung 3 bei der dargestellten Ausführungsform aus zwei über jeweils ein Federelement 4 in Verrastungsrichtung federbelasteten Rasthaken 5 sowie zwei am Kupplungsstecker 2 angeordneten Rastaufnahme 6 zur Aufnahme der mindestens zwei Rasthaken 5 besteht. Bei der dargestellten Ausführungsform sind die Rastaufnahmen 6 als vom Kupplungsstecker 2 radial nach außen abstehende Stege 6 ausgebildet.

Wie aus den Abbildungen ersichtlich, ist die Rastverbindung 3 so ausgebildet, dass die Rasthaken 5 an der Kupplungsaufnahme 1 und die Rastaufnahmen 6 am Kupplungsstecker 2 angeordnet sind.

Derartige Kupplungsmechanismen werden vorzugsweise verwendet, um zwei medizinische Instrumente, beispielsweise ein endoskopisches Instrument und eine Endoskop-Optik, gas- und flüssigkeitsdicht miteinander zu verbinden.

Wie insbesondere aus der Explosionszeichnung gemäß Fig. 1 ersichtlich, besteht die Kupplungsaufnahme 1 im Wesentlichen aus einer Innenhülse 7, einer von der Kupplungsseite her auf die Innenhülse 7 aufsetzbaren und diese koaxial umgebenden Außenhülse 8, einem vom freien unteren Ende auf die Innenhülse 7 aufschiebbaren und diese koaxial umgebenden Federelement 9 sowie einem ebenfalls vom freien unteren Ende auf die Innenhülse 7 aufschiebbaren Ring 10. Beidseitig abgeschlossen wird diese mehrteilig aufgebaute Kupplungsaufnahme 1 über eine auf die Außenhülse 8 aufsetzbare obere Abdeckkappe 11 sowie eine vom freien unteren Ende auf die Innenhülse 7 aufschiebbare untere Abschlusskappe 12.

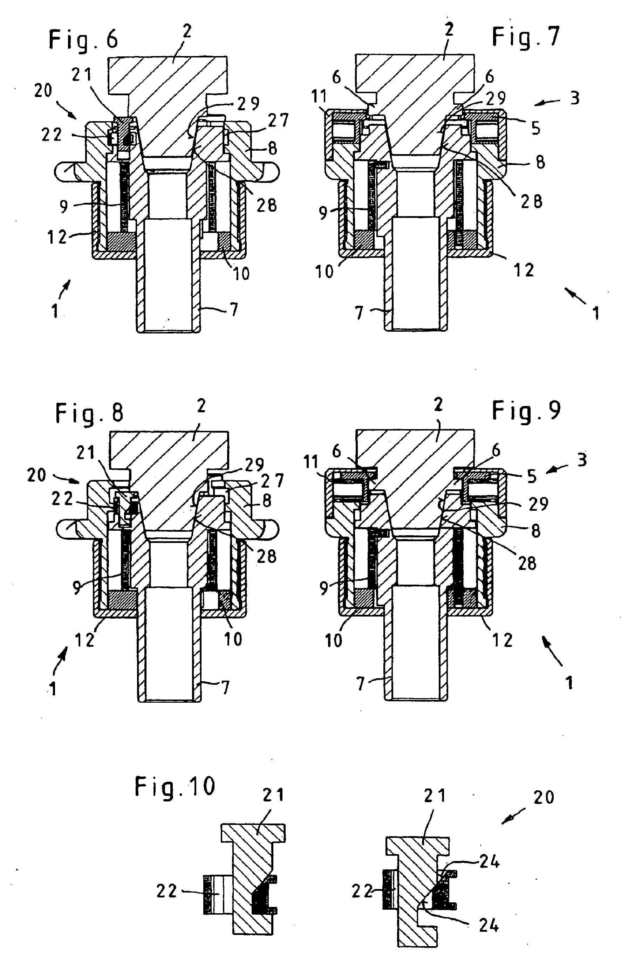

Wie weiterhin aus Fig. 1 in Zusammenschau mit den Abbildungen Fig. 7 und 9 ersichtlich, sind die Innenhülse 7 und die Außenhülse 8 gegeneinander über das als Druck- und Torsionsfeder ausgebildete Federelement 9 verdrehbar miteinander gekoppelt. Zu diesem Zweck sind am Federelement 9 zwei Zapfen 13 angeformt, über die das Federelement 9 einerseits in einer Zapfenaufnahme 14 an der Innenhülse 7 und andererseits in einer Zapfenaufnahme 14 am Ring 10 festlegbar ist. Weiterhin sind am Ring 10 radial nach außen abstehende Vorsprünge 15 ausgebildet, die im zusammengesetzten Zustand der Kupplungsaufnahme 1 in korrespondierende Ausnehmungen 16 in der Außenhülse 8 eingreifen.

Das eigentliche Koppeln der Kupplungsaufnahme 1 mit dem Kupplungsstecker 2 mittels der Rastverbindung 3 erfolgt über an den Rasthaken 5 ausgebildete, nach innen weisende Rastnasen 17, die im verrasteten Zustand die als Stege 6 ausgebildete Rastaufnahme 6 des Kupplungssteckers 2 hintergreifen, wie dies den Abbildungen Fig. 7, 9 und 12 zu entnehmen ist.

Um die Rasthaken zwischen einer die Kupplungsteile 1 und 2 miteinander verrastenden und einer diese wieder freigebenden Stellung verstellen zu können, sind die Rasthaken 5 radial verschiebbar so in Führungen 18 der Außenhülse 8 gelagert, dass sich die die Rasthaken 5 in Verrastungsrichtung vorspannenden Federelemente 4 einerseits an der Innenseite der Abdeckkappe 11 abstützen und andererseits an den Rasthaken 5 anliegen, um diese radial nach innen zu drücken, bis die Rastnasen 17 der Rasthaken 5 im mit dem Kupplungsstecker 2 verrasteten Zustand die Stege 6 des Kupplungssteckers 2 hintergreifen. Die Verschiebung der Rasthaken 5 radial nach innen wird im mit dem Kupplungsstecker 2 verrasteten Zustand dadurch begrenzt, dass die Rasthaken 5 innenseitig an einer Anlagefläche 19 der Innenhülse 7 zur Anlage kommen. Diese Anlagefläche 19 der Innenhülse 7 ist, wie insbesondere aus Fig. 1 ersichtlich als unrunde Kulissenbahn ausgebildet.

Als Besonderheit weist der dargestellte Kupplungsmechanismus einen Sicherungsmechanismus 20 auf, über den die Rastverbindung 3 aktivierbar ist. Der Sicherungsmechanismus 20 dient dazu, ein versehentliches Verrasten der Rastverbindung 3 ohne eingesetztes Instrument zu verhindern. Wie aus den Abbildungen Fig. 1, 3, 5, 6 und 8 sowie insbesondere Fig. 10 ersichtlich, ist der Sicherungsmechanismus 20 bei der dargestellten Ausführungsform als aus einem axial verschiebbaren Stift 21 und einem mit dem Stift 21 in Wirkverbindung stehendes radial verschiebbares Klemmelement 22 ausgebildet, wobei das Klemmelement 22 über ein vorteilhafterweise als Federblech ausgebildetes Federelement 23 in die den Sicherungsmechanismus 20 sperrende Richtung vorgespannt ist.

Um das Zusammenspiel des axial verschiebbaren Stifts 21 mit dem radial verschiebbares Klemmelement 22 zu erleichtern und das Überführen des Sicherungsmechanismus 20 von der die Rastverbindung 3 sperrenden in die die Rastverbindung 3 freigebende Stellung zu vereinfachen, sind an miteinander in Wirkverbindung stehenden Kontaktflächen des Stifts 21 und des Klemmelmenets 22 korrespondierende Anlaufschrägen 24 ausgebildet, die vorzugsweise einen Winkel von 45° aufweisen, wodurch der Stift 21 beim Anlaufen gegen das Klemmelement 22 das Klemmelement 22 automatisch radial nach innen drückt.

Das Trennen der beiden über den Kupplungsmechanismus miteinander verbundenen Bauteile 1 und 2 ist die Rastverbindung 3 über einen mit den Rasthaken 5 in Wirkverbindung stehenden Auslösemechanismus 25 wieder aufhebbar.

Wie insbesondere aus den Abbildungen Fig. 1, 2, 4 sowie 11 und 12 ersichtlich, besteht der Auslösemechanismus 25 aus der als unrunde Kulissenbahn ausgebildeten Anlagefläche 19 der Innenhülse 7 sowie aus an der Außenhülse 8 ausgebildete, radial nach außen vorstehenden Nocken 26, über die die Außenhülse 8 gegenüber der Innenhülse 7 verdrehbar ist. Beim Verdrehen der Außenhülse 8 verfahren die im verrasteten Zustand des Kupplngsmechanismus an der Anlagefläche 19 anliegenden Rasthaken 5 entlang dieser unrunden Kulissenbahn und werden während dieses Verfahrens radial nach außen gedrückt, bis die Rastnasen 17 der Rasthaken 5 die Stege 6 des Kupplungssteckers 2 nicht mehr hintergreifen und die Kopplung der Bauteile 1 und 2 sowie die Innenhülse 7 freigeben.

Der Zusammenbau sowie das Betätigen des Kupplungsmechanismus wird nachfolgend anhand der Abbildungen Fig.1 und 6 bis 9 erläutert:

Im ersten Montageschritt wird der aus dem Stift 21, dem Klemmelement 22 sowie dem Federelement 23 bestehende Sicherungsmechanismus 20 an der Innenhülse 7 festgelegt.

Nachfolgend wird vom kupplungsfernen unteren Ende der Innenhülse 7 her zunächst das als Druck- und Torsionsfeder ausgebildete Federelement 9 auf die Innenhülse 7 aufgeschoben, bis der Zapfen 13 des Federelements 9 in die Zapfenaufnahme 14 der Innenhülse 7 eingreift. Im nächsten Schritt wird ebenfalls vom kupplungsfernen unteren Ende der Innenhülse 7 her der Ring 10 auf die Innenhülse 1 aufgeschoben, bis der Zapfen 13 des Federelements 9 in die Zapfenaufnahme 14 des Rings 10 eingreift.

Auf diese Baugruppe wird nunmehr vom oberen kupplungsseitigen Ende der Innenhülse 7 her die Außenhülse 8 auf die Innenhülse 7 aufgesetzt, bis die am Ring 10 angeformten Vorsprünge 15 in die Ausnehmungen 16 der Außenhülse 8 eingreifen. Zusammengehalten wird diese über das Federelement 9 gegeneinander verdrehbare Kopplung der Innenhülse 7 mit der Außenhülse 8 über die vom freien unteren Ende auf die Innenhülse 7 aufschiebbare und auf die Außenhülse 8 aufschraubbare Abschlusskappe 12.

Im nächsten Montageschritt werden die Rasthaken 5 und die Federelemente 4 in die Führungen 18 der Außenhülse 8 eingesetzt und abschließend vom kopplungsseitigen Ende her die obere Abdeckkappe 11 so auf die Außenhülse 8 aufgesetzt und mit dieser beispielsweise durch Verschweißen verbunden, dass sich die Federelemente 4 rückseitig an der Innenseite der Abdeckkappe 11 abstützen und die Rasthaken 5 radial einwärts gegen die Innenhülse 1 drücken.

In diesem zusammengesetzten Zustand der Kupplungsaufnahme 1 drückt das Federelement 23 des Sicherungsmechanismus das radial verschiebbare Klemmelement 22 radial nach außen in einen Freistich 27 in der Außenhülse 8, wie dies in Fig. 6 dargestellt ist. In dieser Stellung sperrt der Sicherungsmechanismus 20 die Rastverbindung 3 gegen ein versehentliches Verrasten.

Der Kupplungsmechanismus ist nunmehr bereit die verrastende Verbindung zwischen dem Kupplungsstecker 2 in der Kupplungsaufnahme 1 zu ermöglichen. Bei der dargestellten Ausführungsform sind die ineinander zu fügenden Abschnitte des Kupplungssteckers 2 und der Kupplungsaufnahme 1 als Konus 28 und Gegenkonus 29 ausgebildet. Selbstverständlich sind auch andere Formen, wie rohrförmige Kupplungsabschnitte möglich.

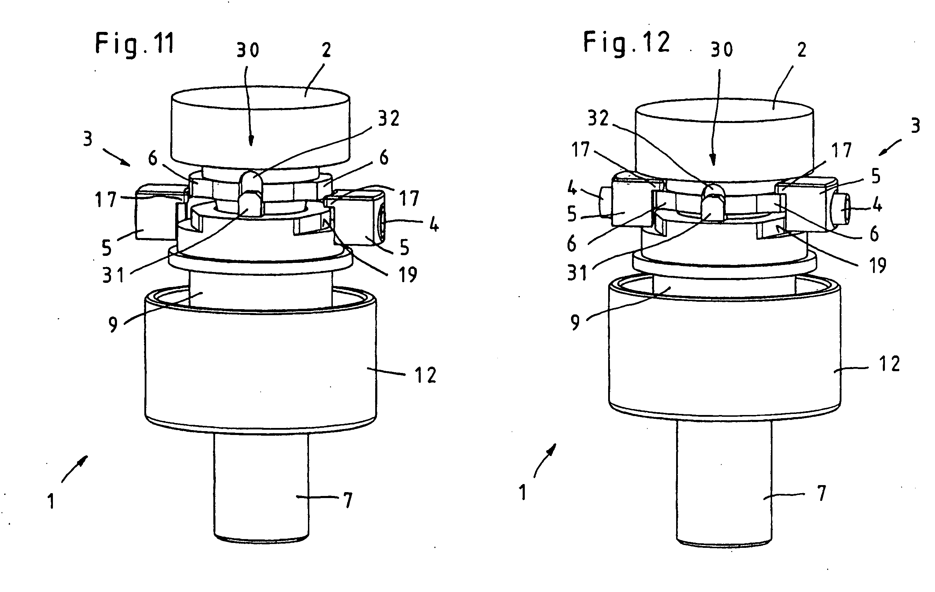

Um eine lagegenaue und verdrehsichere Platzierung der Bauteile Kupplungsaufnahme 1 und Kupplungsstecker 2 zueinander zu gewährleisten, sind, wie aus Fig. 11 und 12 ersichtlich, am Kupplungsstecker 2 und an der Kupplungsaufnahme 1 miteinander korrespondierende Führungs- und/oder Zentrierelemente 30 ausgebildet, die bei der dargestellten Ausführungsform als Zentrierzapfen 31 und Aufnahmenut 32 ausgebildet sind.

Ausgehend von der in Fig. 6 und 11 dargestellten Ausgangslage, in der der Kupplungsstecker 2 und die Kupplungsaufnahme 1 über die Führungs- und/oder Zentrierelemente 28 zueinander ausgerichtet sind und der Kupplungsstecker 2 mit der Unterseite der Rastaufnahme 6 auf dem Stift 21 des Sicherungsmechanismus 20 aufliegt, wird der Kupplungsstecker 2 nachfolgend zur Verrastung mit der Kupplungsaufnahme 1 nach unten in die Kupplungsaufnahme 1 gedrückt. Diese über den Kupplungsstecker 2 auf den Stift 21 des Sicherungsmechanismus 20 ausgeübte axiale Druckkraft bewirkt, dass, wie in Fig. 10 dargestellt, das Klemmelement 22 über die aneinander anliegenden Anlaufschrägen 24 des Stifts 21 und des Klemmelements 22 radial nach innengedrückt wird und den Freistich 27 der Außenhülse 8 wieder freigibt.

Dieses Entriegeln des Sicherungsmechanismus 20 bewirkt, dass der Kupplungsstecker 2 weiter in die Kupplungsaufnahme 1 hineingedrückt werden kann und die Rastverbindung 3 aktiviert wird. Sobald der Kupplungsstecker 2 ausreichend tief in die Kupplungsaufnahme 1 eingetreten ist, werden die über die Federelemente 4 in Verrastungsrichtung vorgespannten Rasthaken 5 radial nach innen gedrückt, bis die Rastnasen 17 der Kupplungsaufnahme 1 die Stege 6 des Kupplungssteckers 2 hintergreifen und den Kupplungsstecker 2 gas- und flüssigkeitsdicht in der Kupplungsaufnahme 1 fixieren.

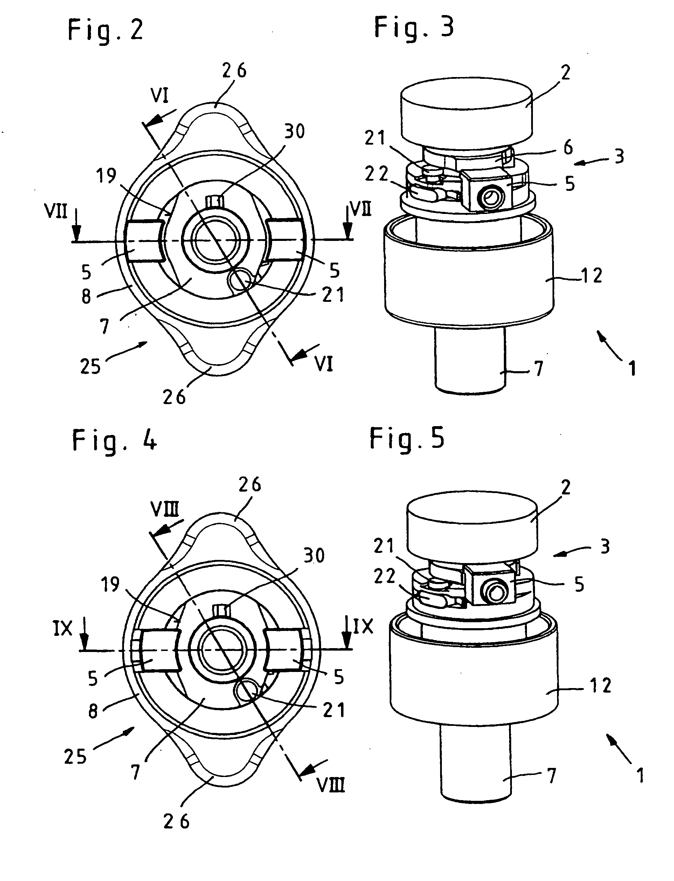

Zum Lösen der Rastverbindung wird die Außenhülse 8 über die beiden insbesondere in Fig. 2 und 4 dargestellten Nocken 26 gegenüber der Innenhülse 7 verdreht, bis die an der Anlagefläche 19 de Innenhülse 1 anliegenden Rasthaken 5 durch die unrunde Kulissenbahn der Anlagefläche 19 radial nach außen gedrückt werden und die Rastnasen 17 der Rasthaken 5 die Stege 6 des Kupplungssteckers 2 treten.

Durch das als Druck- und Torsionsfeder ausgebildete Federelement 9, das einerseits die Gegeneinanderverdrehung von Innenhülse 7 und Außenhülse 8 ermöglicht und andererseits als axialer Toleranzausgleich bei der Verrastung mit dem Kupplungsstecker 2 dient, wird die Außenhülse 8 aufgrund der Torsionskraft automatisch wieder in die Ausgangslage zurück gedreht, sobald die Rastverbindung 3 gelöst wird. Diese Rückdrehung der Außenhülse 8 bewirkt ein axiales Anheben des Kupplungssteckers 2 heraus aus der Kupplungsaufnahme 1.

In diesem entriegelten Zustand drückt das Federelement 23 das Klemmelement 22 des Sicherungsmechanismus 20 wieder radial nach außen in den Freistich 27 der Außenhülse 8, wodurch der Stift 21 wieder die in Fig. 6 dargestellte Position einnimmt und der Sicherungsmechanismus 20 die Rastverbindung 3 sperrt.

Ein solchermaßen ausgestalteter Kupplungsmechanismus zeichnet sich dadurch aus, dass er durch bloßes Zusammendrücken der miteinander zu verbindenden Bauteile 1 und 2 eine sichere sowie gas- und flüssigkeitsdichte Verbindung bewirkt.

**Bezugszeichenliste**

| | | | |
|---|---|---|---|
| 1 | Kupplungsaufnahme | 18 | Führung |
| 2 | Kupplungsstecker | 19 | Anlagefläche |
| 3 | Rastverbindung | 20 | Sicherungsmechanismus |
| 4 | Federelement | 21 | Stift |
| 5 | Rasthaken | 22 | Klemmelement |
| 6 | Rastaufnahme/Steg | 23 | Federelement |
| 7 | Innenhülse | 24 | Anlaufschräge |
| 8 | Außenhülse | 25 | Auslösemechanismus |
| 9 | Federelement | 26 | Nocke |
| 10 | Ring | 27 | Freistich |
| 11 | Abdeckkappe | 28 | Konus |
| 12 | Abschlusskappe | 29 | Gegenkonus |
| 13 | Zapfen | 30 | Führungs- / Zentrierelemente |
| 14 | Zapfenaufnahme | 31 | Zentrierzapfen |
| 15 | Vorsprung | 32 | Aufnahmenut |
| 16 | Ausnehmung | | |
| 17 | Rastnase | | |

## Patentansprüche

1. Kupplungsmechanismus, insbesondere für medizinische Instrumente, mit einem Kupplungsstecker (2) und einer Kupplungsaufnahme (1) zur Aufnahme des Kupplungssteckers (2), wobei die Kupplungsaufnahme (1) und der Kupplungsstecker (2) über mindestens eine federbelastete Rastverbindung (3) aneinander festlegbar sind, wobei die Rastverbindung (3) über einen Sicherungsmechanismus (20) aktivierbar ist, die über den in die Kupplungsaufnahme (1) eingesteckten Kupplungsstecker (2) freigebbar ist,
**dadurch gekennzeichnet,**
**dass** der Sicherungsmechanismus (20) als federbelastetes Rastelement ausgebildet ist, das als von der Rastverbindung (3) getrenntes eigenständiges Bauteil in der Kupplungsaufnahme (1) angeordnet ist.

2. Kupplungsmechanismus nach Anspruch 1, **dadurch gekennzeichnet, dass** das Rastelement aus einem axial verschiebbaren Stift (21) und einem mit dem Stift (21) in Wirkverbindung stehenden radial verschiebbaren Klemmelement (22) besteht, wobei das Klemmelement (22) über ein Federelement (23) in die den Sicherungsmechanismus (20) sperrende Richtung vorgespannt ist.

3. Kupplungsmechanismus nach Anspruch 2, **dadurch gekennzeichnet, dass** am Stift (21) und am Klemmelement (22) miteinander korrespondierende Anlaufschrägen (24) ausgebildet sind.

4. Kupplungsmechanismus nach einem der Ansprüche 1 bis 3, **dadurch gekennzeichnet, dass** die Rastverbindung (3) aus mindestens einem an der Kupplungsaufnahme (1) angeordneten und über mindestens ein Federelement (4) in Verrastungsrichtung federbelasteten Rasthaken (5) sowie mindestens einer am Kupplungsstecker (2) angeordneten Rastaufnahme (6) zur Aufnahme des mindestens einen Rasthakens (5) besteht.

5. Kupplungsmechanismus nach einem der Ansprüche 1 bis 4 **dadurch gekennzeichnet, dass** die Kupplungsaufnahme (1) eine Innenhülse (7) und eine die Innenhülse (7) zumindest teilweise koaxial umgebende Außenhülse (8) umfasst, wobei die Innenhülse (7) und die Außenhülse (8) über ein Federelement (9), insbesondere eine Druckfeder, gegeneinander verdrehbar miteinander gekoppelt sind.

6. Kupplungsmechanismus nach Anspruch 4 oder 5, **dadurch gekennzeichnet, dass** an den Rasthaken (5) nach innen weisende Rastnasen (17) ausgebildet sind, die im mit dem Kupplungsstecker (2) verrasteten Zustand als Stege (6) ausgebildete Rastaufnahmen (6) am Kupplungsstecker (2) hintergreifen.

7. Kupplungsmechanismus nach Anspruch 5 oder 6, **dadurch gekennzeichnet, dass** die Rasthaken (5) radial verschiebbar in der Außenhülse (8) gelagert sind und im mit dem Kupplungsstecker (2) verrasteten Zustand innenseitig an eine Anlagefläche (19) der Innenhülse (7) anliegen.

8. Kupplungsmechanismus nach einem der Ansprüche 1 bis 7, **dadurch gekennzeichnet, dass** die Rastverbindung (3) über einen mit dem mindestens einen Rasthaken (5) in Wirkverbindung stehenden Auslösemechanismus (25) wieder aufhebbar ist.

9. Kupplungsmechanismus nach Anspruch 8, **dadurch gekennzeichnet, dass** zur Ausbildung des Auslösemechanismus (25) die Anlagefläche (19) der Innenhülse (7), an der die Rasthaken (5) im verrasteten Zustand anliegen, als unrunde Kulissenbahn ausgebildet ist.

10. Kupplungsmechanismus nach einem der Ansprüche 5 bis 9**, dadurch gekennzeichnet, dass** an der Außenhülse (8) radial nach außen vorstehende Nocken (26) ausgebildet sind.

11. Kupplungsmechanismus nach einem der Ansprüche 1 bis 10, **dadurch gekennzeichnet, dass** am Kupplungsstecker (2) und an der Kupplungsaufnahme (1) miteinander korrespondierende Führungs- und/oder Zentrierelemente (30) ausgebildet sind.

12. Kupplungsaufnahme zur Aufnahme eines Kupplungssteckers (2) eines Kupplungsmechanismus, insbesondere für medizinische Instrumente, nach einem der Ansprüche 1 bis 11, wobei die Kupplungsaufnahme (1) und der Kupplungsstecker (2) über mindestens eine federbelastete Rastverbindung (3) aneinander festlegbar sind und wobei die Rastverbindung (3) über einen Sicherungsmechanismus (20) aktivierbar ist, die über den in die Kupplungsaufnahme (1) eingesteckten Kupplungsstecker (2) freigebbar ist, **dadurch gekennzeichnet, dass** der Sicherungsmechanismus (20) als federbelastetes Rastelement ausgebildet ist, das als von der Rastverbindung (3) getrenntes eigenständiges Bauteil in der Kupplungsaufnahme (1) angeordnet ist.

## Claims

1. Coupling mechanism, in particular for medical instruments, with a coupling plug (2) and a coupling socket (1) for receiving the coupling plug (2), said coupling socket (1) and said coupling plug (2) being able to be secured to each other via at least one spring-loaded latching connection (3), and said latching connection (3) being able to be activated via a safety mechanism (20) and released via the coupling plug (2) inserted into the coupling socket (1), **characterized in that** the safety mechanism (20) is designed as a spring-loaded latching element which is arranged as an independent component, separate from the latching connection (3), in the coupling socket (1).

2. Coupling mechanism according to Claim 1, **characterized in that** the latching element is composed of an axially movable pin (21) and of a radially movable clamping element (22) operatively connected to the pin (21), said clamping element (22) being pretensioned via a spring element (23) in the direction blocking the safety mechanism (20).

3. Coupling mechanism according to Claim 2, **characterized in that** run-on slopes (24) that correspond to each other are formed on the pin (21) and on the clamping element (22).

4. Coupling mechanism according to one of Claims 1 to 3, **characterized in that** the latching connection (3) is composed of at least one latching hook (5), which is arranged on the coupling socket (1) and is spring-loaded in the locking direction via at least one spring element (4), and of at least one latching receiver (6) which is arranged on the coupling plug (2) and receives the at least one latching hook (5).

5. Coupling mechanism according to one of Claims 1 to 4, **characterized in that** the coupling socket (1) comprises an inner sleeve (7) and an outer sleeve (8) that coaxially surrounds the inner sleeve (7) at least in part, the inner sleeve (7) and the outer sleeve (8) being coupled to each other, in such a way that they can turn relative to each other, via a spring element (9), in particular a compression spring.

6. Coupling mechanism according to Claim 4 or 5, **characterized in that** inwardly directed latching lugs (17) are formed on the latching hooks (5) and, in the state when locked to the coupling plug (2), engage behind latching receivers (6) designed as webs (6) on the coupling plug (2).

7. Coupling mechanism according to Claim 5 or 6, **characterized in that** the latching hooks (5) are mounted in a radially movable manner in the outer sleeve (8) and, in the state when locked to the coupling plug (2), bear inwardly on a contact surface (19) of the inner sleeve (7).

8. Coupling mechanism according to one of Claims 1 to 7, **characterized in that** the latching connection (3) can be released again via a release mechanism (25) operatively connected to the at least one latching hook (5).

9. Coupling mechanism according to Claim 8, **characterized in that**, in order to form the release mechanism (25), the contact surface (19) of the inner sleeve (17), on which the latching hooks (5) bear in the locked state, is designed as an out-of-round guide track.

10. Coupling mechanism according to one of Claims 5 to 9, **characterized in that** radially outwardly projecting cams (26) are formed on the outer sleeve (8).

11. Coupling mechanism according to one of Claims 1 to 10, **characterized in that** mutually corresponding guiding and/or centring elements (30) are formed on the coupling plug (2) and on the coupling socket (1).

12. Coupling socket for receiving a coupling plug (2) of a coupling mechanism, in particular for medical instruments, according to one of Claims 1 to 11, said coupling socket (1) and said coupling plug (2) being able to be secured to each other via at least one spring-loaded latching connection (3), and said latching connection (3) being able to be activated via a safety mechanism (20) and released via the coupling plug (2) inserted into the coupling socket (1), **characterized in that** the safety mechanism (20) is designed as a spring-loaded latching element which is arranged as an independent component, separate from the latching connection (3), in the coupling socket (1).

## Revendications

1. Mécanisme de couplage ou d'accouplement, notamment pour instruments médicaux, comprenant une pièce de couplage mâle (2) et une pièce de couplage femelle (1) destinée à recevoir la pièce de couplage mâle (2), la pièce de couplage femelle (1) et la pièce de couplage mâle (2) pouvant être fixées l'une à l'autre par l'intermédiaire d'au moins un système de liaison par encliquetage (3) sollicité par ressort, et le système de liaison par encliquetage (3) pouvant être rendu actif par l'intermédiaire d'un mécanisme d'arrêt de sécurité (20), qui peut être libéré par l'intermédiaire de la pièce de couplage mâle (2) insérée dans la pièce de couplage femelle (1),
**caractérisé en ce que** le mécanisme d'arrêt de sécurité (20) est réalisé sous la forme d'un élément d'enclenchement sollicité par ressort, qui est agencé, en tant que pièce propre, séparée du système de liaison par encliquetage (3), dans la pièce de couplage femelle (1).

2. Mécanisme de couplage selon la revendication 1, **caractérisé en ce que** l'élément d'enclenchement est constitué par une broche (21) axialement coulissante et par un élément de serrage (22) pouvant coulisser radialement et en liaison interactive avec la broche (21), la pièce de serrage (22) étant précontrainte par un élément de ressort (23), dans la direction verrouillant le mécanisme d'arrêt de sécurité (20).

3. Mécanisme de couplage selon la revendication 2, **caractérisé en ce que** sur la broche (21) et sur l'élément de serrage (22) sont formées des surfaces en rampe (24) mutuellement correspondantes.

4. Mécanisme de couplage selon l'une des revendications 1 à 3, **caractérisé en ce que** le système de liaison par encliquetage (3) est constitué par au moins un crochet d'encliquetage (5) agencé sur la pièce de couplage femelle (1) et sollicité par ressort dans la direction d'encliquetage par l'intermédiaire d'au moins un élément de ressort (4), ainsi que par au moins un logement de réception d'encliquetage (6) agencé sur la pièce de couplage mâle (2) et destiné à recevoir ledit au moins un crochet d'encliquetage (5).

5. Mécanisme de couplage selon l'une des revendications 1 à 4, **caractérisé en ce que** la pièce de couplage femelle (1) comprend une douille intérieure (7) et une douille extérieure (8) entourant coaxialement au moins partiellement la douille intérieure (7), la douille intérieure (7) et la douille extérieure (8) étant couplées l'une à l'autre par l'intermédiaire d'un élément de ressort (9), notamment un ressort de compression, de manière à pouvoir tourner l'une par rapport à l'autre.

6. Mécanisme de couplage selon la revendication 4 ou la revendication 5, **caractérisé en ce que** sur les crochets d'encliquetage (5) sont formés des talons d'encliquetage (17) dirigés vers l'intérieur et qui, dans l'état encliqueté avec la pièce de couplage mâle (2), viennent s'engager derrière des logements de réception d'encliquetage (6) réalisés en tant que nervures (6) sur la pièce de couplage mâle (2).

7. Mécanisme de couplage selon la revendication 5 ou la revendication 6, **caractérisé en ce que** les crochets d'encliquetage (5) sont montés de manière radialement coulissante dans la douille extérieure (8), et, dans l'état encliqueté avec la pièce de couplage mâle (2), s'appuient du côté intérieur, sur une surface d'appui (19) de la douille intérieure (7).

8. Mécanisme de couplage selon l'une des revendications 1 à 7, **caractérisé en ce que** le système de liaison par encliquetage (3) peut à nouveau être désolidarisé par l'intermédiaire d'un mécanisme de déclenchement (25) en liaison interactive avec ledit au moins un crochet d'encliquetage (5).

9. Mécanisme de couplage selon la revendication 8, **caractérisé en ce que** pour la réalisation du mécanisme de déclenchement (25), la surface d'appui (19) de la douille intérieure (7), contre laquelle s'appuient les crochets d'encliquetage (5) dans l'état encliqueté, est conçue sous forme de voie de coulissement non circulaire.

10. Mécanisme de couplage selon l'une des revendications 5 à 9, **caractérisé en ce que** sur la douille extérieure (8) sont formées des protubérances (26) faisant saillie radialement vers l'extérieur.

11. Mécanisme de couplage selon l'une des revendications 1 à 10, **caractérisé en ce que** sur la pièce de couplage mâle (2) et sur la pièce de couplage femelle (1) sont formés des éléments de guidage et/ou de centrage (30) mutuellement correspondants.

12. Pièce de couplage femelle destinée à recevoir une pièce de couplage mâle (2) d'un mécanisme de couplage, notamment pour instruments médicaux, selon l'une des revendications 1 à 11, la pièce de couplage femelle (1) et la pièce de couplage mâle (2) pouvant être fixées l'une à l'autre par l'intermédiaire d'au moins un système de liaison par encliquetage (3) sollicité par ressort, et le système de liaison par encliquetage (3) pouvant être rendu actif par l'intermédiaire d'un mécanisme d'arrêt de sécurité (20), qui peut être libéré par l'intermédiaire de la pièce de couplage mâle (2) insérée dans la pièce de couplage femelle (1), **caractérisé en ce que** le mécanisme d'arrêt de sécurité (20) est réalisé sous la forme d'un élément d'enclenchement sollicité par ressort, qui est agencé, en tant que pièce propre, séparée du système de liaison par encliquetage (3), dans la pièce de couplage femelle (1).
